# EUROPEAN PATENT SPECIFICATION

(11) **EP 3 754 323 B1**
(45) Date of publication and mention of the grant of the patent: **23.08.2023**
(21) Application number: 19180913.6
(22) Date of filing: 18.06.2019
(51) Int. Cl.: G01N 15/14, G01N 21/65, G01N 33/00, G01N 1/00, G01N 15/00

(54) **AUTOMATIC SPORE TRAP**
AUTOMATISCHE SPORENFALLE
PIÈGE DE SPORE AUTOMATIQUE

(43) Date of publication of application: 23.12.2020
(73) Proprietor: Plair SA, 1258 Perly (CH)
(72) Inventor: KISELEV, Denis, 1258 Perly (CH); KISELEVA, Svetlana, 1258 Perly (CH)
(74) Representative: KATZAROV S.A.

(56) References cited:
- WO-A2-03/061586
- CN-A- 109 781 982
- GB-A- 2 387 902
- US-A1- 2003 098 422
- US-A1- 2014 004 559

## Description

### Technical Field

The present invention relates to spore monitoring and more particularly to a device and a method for spore trapping and monitoring.

### Background of the art

Fungal spores, particular those contaminating leaves and fruits in arboriculture, horticulture and viticulture, have enormous economic impact. They affect quality of crops and losses due to certain pathogens can be up to 80% if not properly prevented.

In order to prevent such fungal spore proliferation, it is necessary to monitor the spore activity.

Current methods of spore monitoring can be divided in two main groups consisting in predictive methods and post-analysis (sampling) methods.

The first group contains prediction models, generally distributed as software, using current meteorological values, historical data and sophisticated algorithms. These models provide per hour prediction of eventual ascospore release due to favorable conditions and maturity. Different software solutions have been elaborated and are wildly sold around the world. The main advantage of these solutions is their fast and generally overprotective response. Indeed, such software programs will in general predict spore emission whenever the meteorological conditions are suitable, for example right after the rain. This means that farmer that follows the forecast of such models is most probably secured from having plant disease such as apple scab. In the same time, the over-predictive aspect of these software solutions leads to the fact that arbori-, horti- and viti-farmers have to over-treat their plantations with fertilizers and pesticides as there is no reliable way to verify rapidly whether the alert was true or false. This leads to severe over-treatment which is harmful for the plants as well as for the final consumer.

The second group of methods combines all instruments that sample air on a medium that will be further analyzed by manual or automatic approaches. An example of such method is a classical Marchi spore trap that has a mechanism that exposes a scotch tape to the air flow, which then gets analyzed under the microscope by a qualified person. Another example is lab-on-chip or molecular methods that collect air on a sampled medium or in water and then analyze it by PCR, DNA sequencing, targeted markers and fluorescence detections, etc.

US patent (US 2014/004559A1) discloses a system for trapping airborne particles such as pollens, fungal spores, bacteria, or viruses and measuring optical spectra thereof.

All these methods require sample collection, an operator, and post analysis, leading to delayed response. Moreover, running such methods to monitor ascospore and conidia emission continuously or even during days with favorable meteorological conditions makes them highly expensive and not scalable.

There is therefore a need for such a new reliable and responsive spore monitoring device and method.

In this regard, a primary object of the invention is to solve the above-mentioned problems and more particularly to provide a device and a method providing a rapid and reliable spore monitoring adapted to provide a reliable measurement within a short period of time thereby preventing both overtreatment and proliferation of spores.

### Summary of the invention

The above problems are solved by the present invention. More particularly, the method and device of the present invention is adapted for automatic tracking of relevant fungal spores and alerting concerned parties about excessive level of concentration of said relevant fungal spores. This permits better prevention of plant disease such as apple scab, downy and powdery mildew, botrytis, or other fungal pathogens.

In this regard, a first aspect of the invention is measurement device that comprises contaminated leaves of the agriculture plant of interest (tree or other types), an aspiration mechanism with a pump, a first light source for inducing light scattering on individual airborne particles, preferably laser or laser diode, multiangle light scattering detector, acquiring signals coupled with reading electronics, triggering a second light source to excite induced fluorescence, preferably UV LED(s) or laser(s), and spectrally resolved light sensor coupled with reading electronics.

Furthermore, the device of the invention comprises a warning system adapted to send a signal to a user that spores have been detected when spores are detected. The device of the present invention is defined in claim 1.

Preferably, the contaminated leaves are leaves which have fell from the plant of interest in the past, preferably from the previous season, so they will have the same fungus and will emit the same spore than the ones in the field with the same meteorological conditions.

When this device is in use, preferably in the field of interest, or next to it so as to experience the same meteorological conditions, and when the device will detect that the contaminated leaves, or more particularly the fungus on the contaminated leaves, within the device forms and emits spores, it will warn the user that spores have been detected in the device and that, in consequence of a risk that the same is happening in the field of interest.

These leaves will have to be laid preferably flat next to each other, even more preferably covering a surface of 1 x 1 m or more, and the measurement part should be place on top of the leaves or right next to them.

Advantageously, the leaves should be placed between small section metal grids like mosquito net to prevent ground worms and insect from destroying them.

Preferably, the multi-angle light scattering detector should cover at least 4 scattering angles.

According to a preferred embodiment, the multi-angle light scattering detector should acquire traces of scattering in timely matter, meaning that passage of each particle should be recorded and resolved temporally. The acquisition speed of the electronics should be at least 1 Mega sample per second and per channel.

Advantageously, the spectrally resolved light sensor covers at least 4 wavelength ranges (fluorescence bands).

A second aspect of the invention is a method of providing a user with an information that spores are emitting in a field of interest consisting in placing the device of the first aspect in or near the field of interest such that it experiences the same meteorological conditions as the field, running in real time the detection of the spores, and upon detection of the spores of interest, sending a warning or information signal to a user indicating the same.

A third aspect of the invention relates to the use of the device of the first aspect consisting in placing it in or near the field of interest such that it experiences the same meteorological conditions as the field, running in real time the detection of the spores, and upon detection of the spores of interest, sending a warning or information signal to a user indicating the same.

### Brief description of the drawings

Further particular advantages and features of the invention will become more apparent from the following non-limitative description of at least one embodiment of the invention which will refer to the accompanying drawings, wherein
- Figure 1 represents an automatic spore trap according to a first embodiment of the present invention,
- Figure 2 represents an automatic spore trap according to a second embodiment of the present invention, and
- Figure 3 represents an automatic spore trap according to a third embodiment of the present invention.

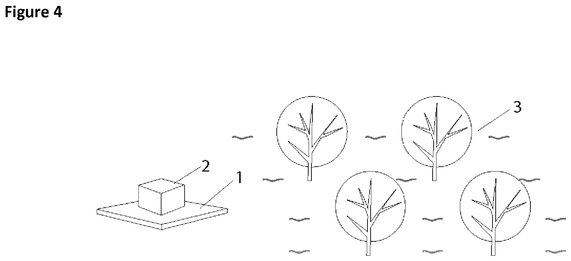

- Figure 4 represents the device of the present invention in use in or near a field of interest.

### Detailed description of the invention

The present detailed description is intended to illustrate the invention in a non-limitative manner since any feature of an embodiment may be combined with any other feature of a different embodiment in an advantageous manner.

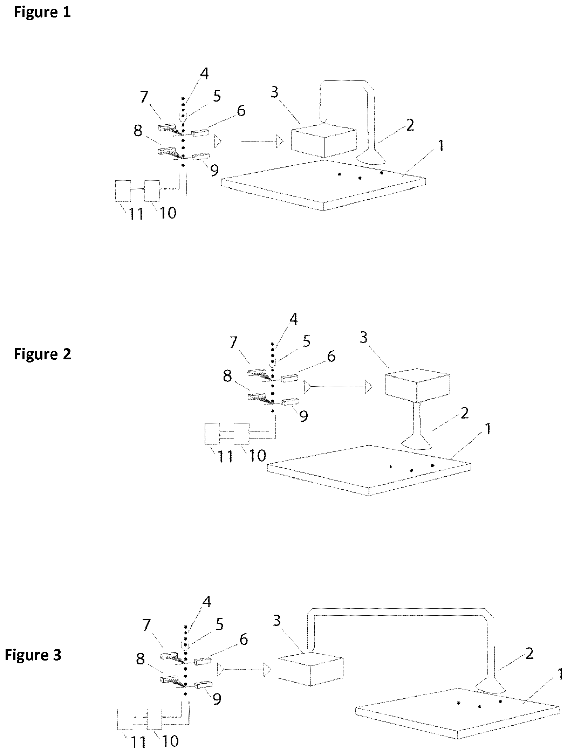

Figure 1 shows the first aspect of the invention which is a preferred embodiment of the present invention relating to an automatic spore trap containing a reservoir of contaminated leaves 1 of the plant of interest, collected during previous year, preferably previous season, and kept during the season in proximity of plants or plantations that need to be protected by the trap.

The preferable quantity of leaves should be at least one hundred, with each or most of leaves presenting traces of disease (characteristic marks, color changes, white net-like coating, etc.). The leaves should be placed between two small section grids preventing penetration of insects.

The trap also contains an electronic device 3 that is placed on the leaves as shown in figures 1 and 2 or right next to them as shown in figure 3. This device contains an inlet or aspiration tube 2, an optical chamber composed of two measurement stages: light scattering and fluorescence, a high purity filter 10 and an air pump 11. The inlet or aspiration tube aspires air in a proximity of the leave reservoir or directly on top of the leaves thanks to the air pump. The geometry of such a tube or inlet and input air flow is such that it provides a way across with a significant quantity of spores can pass through it without stacking or bouncing back. The required efficiency i.e. pass through to total ratio, is to be defined depending on the pathogen fungal spore that is to be measured, typical number of projected ascospores or/and conidia and signal-to-noise ratio of the detection chamber.

The detection chamber is the optical device that has air aspired by the inlet or tube passed through and employs one or multiple lasers or/and flash lamps, LEDs, laser diode, or other optical sources 6 and 9, and one or multiple optical detectors such photomultipliers, photodiodes, avalanche photodiodes, or others 7 and 8 to induce and detect at least one of light scattering intensity, angular patterns, timely-resolved signal or any combination of these or / and with at least one or fluorescence intensity, spectrum, decay or any combination of these.

Each individual particle passing through the system is exposed to both light source(s) inducing light scattering and light source(s) inducing fluorescence. The light scattering patterns and fluorescence spectra are acquired then and combined into a set of parameters such as light scattering patterns, fluorescence spectra, fluorescence decay curves, etc. These parameters are then used form a fingerprint of each particle. The identification of the pathogen under question is done by applying a specially adapted machine learning algorithms, such as artificial neural network, gradient boosted decision trees, random forest, etc., that compares typical signal from already known pathogen(s) with currently detected particles. To learn about known pathogens, the device must be calibrated: exposed to a given pathogen with low background of other particles. The machine learning algorithms are typically of type of classifiers, meaning they attribute labels to unknown raw data, and trained on fingerprints of such calibrations

Advantageously, the device further comprises a warning system adapted to send a signal to a user that spores have been detected when spores are detected.

As explained above, the contaminated leaves are preferably leaves which have fell from the plant of interest in the past, preferably from the previous season, so they will have the same fungus and will emit the same spore than the ones in the field with the same meteorological conditions. Therefore, when this device is in use, preferably in the field of interest, or next to it so as to experience the same meteorological conditions, and when the device will detect that the contaminated leaves, or more particularly the fungus on the contaminated leaves, within the device forms and emits spores thanks to the above described detection chamber, it will warn the user that spores have been detected in the device and that, in consequence the same is happening in the field of interest.

The reporting of the results is done almost instantaneously. The results are qualified by standard statistical values like rate of false positives, false negatives, precision and recall. The results are continuously transmitted to a remote user interface such as on-line dashboard or mobile application. The alert levels are defined depending on the type of pathogen, and typical concentrations leading to contamination.

The invention also relates to a method of providing a user with an information that spores are emitting in a field of interest consisting in placing the above device in or near the field of interest such that it experiences the same meteorological conditions as the field, running in real time the detection of the spores, and upon detection of the spores of interest, sending a warning or information signal to a user indicating the same.

The invention also relates to the use of the above device consisting in placing it in or near the field of interest such that it experiences the same meteorological conditions as the field, running in real time the detection of the spores, and upon detection of the spores of interest, sending a warning or information signal to a user indicating the same.

## Claims

1. Spore trap device comprising a reservoir for receiving contaminated leaves of a field of interest, and being adapted to be placed in or near said field of interest so as to experience the same meteorological conditions as said field of interest
and further comprising an electronic detection device containing an aspiration tube, an optical chamber, a high purity filter and an air pump and **characterized in that** the inlet of the aspiration tube is positioned to aspire air on top of the contaminated leaves and send it to the optical chamber where the optical chamber comprises one or more optical sources and one or multiple optical detectors adapted to detect at least one of light scattering intensity, angular patterns, timely-resolved signal or any combination of these as well as at least one or fluorescence intensity, spectrum, decay or any combination of these caused by the spores and wherein
the device further comprises a warning system adapted to send a signal to a user that spores have been detected when spores are detected in the electronic detection device thereby indicating that the same are present in the field of interest.

2. Spore trap device according to claim 1, **characterized in that** the geometry of said a aspiration tube is such that it provides a way across which a significant quantity of spores can pass through it without stacking or bouncing back depending on the pathogen spore that is to be measured, the typical number of projected ascospores and signal-to-noise ratio of the detection chamber.

3. Spore trap device to claim 1 to 2, **characterized in that** the one or more optical sources comprise one or multiple lasers, flash lamps, LEDs and/or laser diode.

4. Spore trap device according to any one of claims 1 to 3, **characterized in that** the one or multiple optical detector comprises one or multiple optical detectors such as photomultipliers, photodiodes and/or avalanche photodiodes.

5. Spore trap device according to any one of claims 1 to 4, **characterized in that** the leaves are laid flat next to each other, covering a surface of 1 x 1 m or more.

6. Spore trap device according to any one of claims 1 to 5, **characterized in that** the leaves are placed between small section metal grids.

7. Spore trap device according to any one of claims 1 to 6, **characterized in that** the multi-angle light scattering detector covers at least 4 scattering angles.

8. Spore trap device according to any one of claims 1 to 7, **characterized in that** the multi-angle light scattering detector acquires traces of scattering in timely matter such that passage of each particle is recorded and resolved temporally.

9. Spore trap device according to any one of claims 1 to 8, **characterized in that** the acquisition speed of the electronics should be at least 1 Mega sample per second and per channel.

10. Spore trap device according to any one of claims 1 to 9, **characterized in that** the spectrally resolved light sensor covers at least 4 wavelength ranges (fluorescence bands).

11. Method of providing a user with an information that spores are emitting in a field of interest consisting in placing the device of any one of claims 1 to 10 in or near the field of interest such that it experiences the same meteorological conditions as the field, running in real time the detection of the spores, and upon detection of the spores of interest, sending a warning or information signal to a user indicating the same.

12. Use of the device of any one of claims 1 to 10 consisting in placing it in or near the field of interest such that it experiences the same meteorological conditions as the field, running in real time the detection of the spores, and upon detection of the spores of interest, sending a warning or information signal to a user indicating the same.

## Patentansprüche

1. Sporenfallenvorrichtung, die einen Behälter zur Aufnahme von kontaminierten Blättern eines Feldes von Interesse umfasst und dazu geeignet ist, in dem oder in der Nähe des Feldes von Interesse platziert zu werden, um dieselben meteorologischen Bedingungen wie das Feld von Interesse zu erfahren, und ferner eine elektronische Erfassungsvorrichtung umfasst, die ein Saugrohr, eine optische Kammer, einen Hochreinfilter und eine Luftpumpe umfasst, und **dadurch gekennzeichnet, dass** der Einlass des Saugrohrs so positioniert ist, um Luft über den kontaminierten Blättern anzusaugen und sie an die optische Kammer zu schicken, und die optische Kammer eine oder mehrere optische Quellen und einen oder mehrere optische Detektoren umfasst, die dazu geeignet sind, zumindest eines von Lichtstreuungsintensität, Winkelmustern, zeitaufgelösten Signalen oder eine beliebige Kombination davon zu erfassen, sowie zumindest eines von Fluoreszenzintensität, Spektrum, Abschwächung oder eine beliebige Kombination davon, das durch die Sporen verursacht wird, und wobei die Vorrichtung ferner ein Warnsystem umfasst, das dazu geeignet ist, ein Signal an einen Benutzer zu senden, dass in der elektronischen Erfassungsvorrichtung Sporen erfasst wurden, wodurch angezeigt wird, dass dieselben in dem Feld von Interesse vorhanden sind.

2. Sporenfallenvorrichtung nach Anspruch 1, **dadurch gekennzeichnet, dass** die Geometrie des Saugrohrs derart ist, dass es einen Weg bereitstellt, über welchen, in Abhängigkeit von den pathogenen Sporen, die gemessen werden sollen, der typischen Anzahl von gestreuten Ascosporen und dem Signal-Rausch-Verhältnis der Erfassungskammer, eine signifikante Menge von Sporen passieren können, ohne hängenzubleiben oder zurückzuprallen.

3. Sporenfallenvorrichtung nach Anspruch 1 bis 2, **dadurch gekennzeichnet, dass** die eine oder die mehreren optischen Quellen einen oder mehrere Laser, Blitzlichter, LEDs und/oder Laserdioden umfassen.

4. Sporenfallenvorrichtung nach einem der Ansprüche 1 bis 3, **dadurch gekennzeichnet, dass** der eine oder die mehreren optischen Detektoren einen oder mehrere optische Detektoren wie etwa Fotomultiplikatoren, Fotodioden und/oder Avalanche-Fotodioden umfassen.

5. Sporenfallenvorrichtung nach einem der Ansprüche 1 bis 4, **dadurch gekennzeichnet, dass** die Blätter flach nebeneinander abgelegt werden, wobei sie eine Oberfläche von 1 x 1 m oder mehr bedecken.

6. Sporenfallenvorrichtung nach einem der Ansprüche 1 bis 5, **dadurch gekennzeichnet, dass** die Blätter zwischen Metallgittern mit kleinen Sektionen platziert sind.

7. Sporenfallenvorrichtung nach einem der Ansprüche 1 bis 6, **dadurch gekennzeichnet, dass** der Mehrwinkel-Lichtstreuungsdetektor zumindest 4 Streuungswinkel abdeckt.

8. Sporenfallenvorrichtung nach einem der Ansprüche 1 bis 7, **dadurch gekennzeichnet, dass** der Mehrwinkel-Lichtstreuungsdetektor Spuren der Streuung zeitnah erfasst, so dass der Durchgang eines jeden Partikels aufgezeichnet und zeitlich aufgelöst wird.

9. Sporenfallenvorrichtung nach einem der Ansprüche 1 bis 8, **dadurch gekennzeichnet, dass** die Erfassungsgeschwindigkeit der Elektronik zumindest 1 Megasample pro Sekunde und pro Kanal betragen sollte.

10. Sporenfallenvorrichtung nach einem der Ansprüche 1 bis 9, **dadurch gekennzeichnet, dass** der spektral aufgelöste Lichtsensor zumindest 4 Wellenlängenbereiche (Fluoreszenzbänder) abdeckt.

11. Verfahren, um einem Benutzer Informationen darüber bereitzustellen, dass in einem Feld von Interesse Sporen abgegeben werden, wobei das Verfahren darin besteht, die Vorrichtung nach einem der Ansprüche 1 bis 10 in dem oder in der Nähe des Feldes von Interesse zu platzieren, so dass sie dieselben meteorologischen Bedingungen erfährt wie das Feld, die Erfassung der Sporen in Echtzeit durchzuführen, und bei Erfassung der Sporen von Interesse ein Warn- oder Informationssignal, das darauf hinweist, an einen Benutzer zu senden.

12. Verwendung der Vorrichtung nach einem der Ansprüche 1 bis 10, die darin besteht, es in dem oder in der Nähe des Felds von Interesse zu platzieren, so dass sie dieselben meteorologischen Bedingungen erfährt wie das Feld, die Erfassung der Sporen in Echtzeit durchzuführen, und bei Erfassung der Sporen von Interesse ein Warn- oder Informationssignal, das darauf hinweist, an einen Benutzer zu senden.

## Revendications

1. Dispositif de piégeage de spores comprenant un réservoir destiné à recevoir des feuilles contaminées d'un champ d'intérêt, et étant adapté pour être placé dans ou à proximité dudit champ d'intérêt de manière à être soumis aux mêmes conditions météorologiques que ledit champ d'intérêt
et comprenant en outre un dispositif de détection électronique contenant un tube d'aspiration, une chambre optique, un filtre de haute pureté et une pompe à air et **caractérisé en ce que** l'entrée du tube d'aspiration est positionnée pour aspirer l'air au-dessus des feuilles contaminées et l'envoyer à la chambre optique où la chambre optique comprend une ou plusieurs sources optiques et un ou plusieurs détecteurs optiques adaptés pour détecter au moins l'un parmi une intensité de diffusion de lumière, des motifs angulaires, un signal résolu en temps opportun ou toute combinaison de ceux-ci ainsi qu'au moins l'un parmi une intensité de fluorescence, un spectre, une décroissance ou toute combinaison de ceux-ci causés par les spores et dans lequel
le dispositif comprend en outre un système d'avertissement adapté pour envoyer un signal à un utilisateur indiquant que des spores ont été détectées lorsque des spores sont détectées dans le dispositif de détection électronique, ce qui indique que celles-ci sont présentes dans le champ d'intérêt.

2. Dispositif de piégeage de spores selon la revendication 1, **caractérisé en ce que** la géométrie dudit tube d'aspiration est telle qu'elle fournit un passage à travers lequel une quantité significative de spores peut passer sans empilement ni rebond en fonction de la spore pathogène qui doit être mesurée, du nombre typique d'ascospores projetées et du rapport signal sur bruit de la chambre de détection.

3. Dispositif de piégeage de spores selon les revendications 1 et 2, **caractérisé en ce que** la ou les plusieurs sources optiques comprennent un ou plusieurs lasers, lampes éclair, LED et/ou diodes laser.

4. Dispositif de piégeage de spores selon l'une quelconque des revendications 1 à 3, **caractérisé en ce que** le ou les plusieurs détecteurs optiques comprennent un ou plusieurs détecteurs optiques tels que des photomultiplicateurs, des photodiodes et/ou des photodiodes à avalanche.

5. Dispositif de piégeage de spores selon l'une quelconque des revendications 1 à 4, **caractérisé en ce que** les feuilles sont posées à plat les unes à côté des autres, couvrant une surface de 1 x 1 m ou plus.

6. Dispositif de piégeage de spores selon l'une quelconque des revendications 1 à 5, **caractérisé en ce que** les feuilles sont placées entre des grilles métalliques de petite section.

7. Dispositif de piégeage de spores selon l'une quelconque des revendications 1 à 6, **caractérisé en ce que** le détecteur de diffusion de lumière multi-angle couvre au moins 4 angles de diffusion.

8. Dispositif de piégeage de spores selon l'une quelconque des revendications 1 à 7, **caractérisé en ce que** le détecteur de diffusion de lumière multi-angle acquiert des traces de diffusion en temps opportun, de sorte que le passage de chaque particule soit enregistré et résolu dans le temps.

9. Dispositif de piégeage de spores selon l'une quelconque des revendications 1 à 8, **caractérisé en ce que** la vitesse d'acquisition des composants électroniques doit être d'au moins 1 méga échantillon par seconde et par canal.

10. Dispositif de piégeage de spores selon l'une quelconque des revendications 1 à 9, **caractérisé en ce que** le capteur de lumière à résolution spectrale couvre au moins 4 gammes de longueurs d'onde (bandes fluorescentes).

11. Procédé pour fournir à un utilisateur une information selon laquelle des spores sont émises dans un champ d'intérêt consistant à placer le dispositif de l'une quelconque des revendications 1 à 10 dans ou à proximité du champ d'intérêt de sorte qu'il soit soumis aux mêmes conditions météorologiques que le champ, à exécuter en temps réel la détection des spores, et lors de la détection des spores d'intérêt, à envoyer un signal d'avertissement ou d'information à un utilisateur indiquant la même chose.

12. Utilisation du dispositif de l'une quelconque des revendications 1 à 10 consistant à le placer dans ou à proximité du champ d'intérêt de sorte qu'il soit soumis aux mêmes conditions météorologiques que le champ, à exécuter en temps réel la détection des spores et, lors de la détection des spores d'intérêt, à envoyer un signal d'avertissement ou d'information à un utilisateur indiquant la même chose.
